# EUROPEAN PATENT SPECIFICATION

(11) **EP 3 750 584 B1**
(45) Date of publication and mention of the grant of the patent: **15.11.2023**
(21) Application number: 19177739.0
(22) Date of filing: 31.05.2019
(51) Int. Cl.: A61M 16/00

(54) **ACTIVE AND PASSIVE HUMIDIFICATION DEVICE FOR MOUNTING IN A PATIENT VENTILATION CIRCUIT**
AKTIVE UND PASSIVE BEFEUCHTUNGSVORRICHTUNG ZUR MONTAGE IN EINEM PATIENTENBEATMUNGSKREISLAUF
DISPOSITIF D'HUMIDIFICATION ACTIVE ET PASSIVE POUR LE MONTAGE DANS UN CIRCUIT DE VENTILATION D'UN PATIENT

(43) Date of publication of application: 16.12.2020
(73) Proprietor: Sedana Medical Limited, Two Mile House, Kildare Naas (IE)
(72) Inventor: FARRELL, Ronald Martin, Naas, Kildare (IE); CAREY, Pauric, Carbury, Kildare (IE); HENNESSY, Harry, Carlow (IE)
(74) Representative: Schütte, Gearoid

(56) References cited:
- WO-A1-2015/013761
- WO-A1-2018/035579
- WO-A1-2018/060525

## Description

### Introduction

This invention relates to a device for use in the critical care ventilation of patients.

### Background to the Invention

It is necessary that mechanically ventilated patients have heat and moisture supplied/provided to their breathing gas otherwise complications will occur. Initial methods of humidification were based on a device for heating or bubbling water in order to create a vapour in the breathing stream which was referred to as active humidification. Active humidification has been in use for over 60 years and is considered to be very effective for patients in that it supplies copious amounts of moisture and is claimed to match the physiological humidification of the human body in that it can reach temperatures of 37°C and deliver 44 mg H₂O/I of moisture. Intensive testing has shown that popular known active humidification devices do not reach the claimed levels of 44 mg H₂O/I at 37°C at the patient. Because of its perceived capability of delivering 44 mg H₂O/I at 37 °C active humidification is the preferred method used by many physicians but is also believed to be a necessity for certain indications such as when patients are on long term sedation, for paediatric patients or where the patient has poor oxygen uptake or tenacious secretions. Active humidification has the drawback of requiring bulky vapour generating equipment which is mounted on the ventilator with additional tubing and heated breathing tubes in order to prevent moisture build-up and pooling.

Passive humidification was effectively introduced some 30 years ago and involves a plastic enclosure which contains special filter material designed to capture and reflect the heat and moisture from the patient. The passive heat and moisture exchanger (HME) is placed in the breathing circuit between the Y-piece and the endotracheal tube (ET-tube) where it captures the heat and moisture from the patients' exhaled breath and releases it to the patient on inspiration.

The HME has the advantage of compactness with no additional equipment required. HME's operate at about 70 to 80% efficiency and typically return about 33 mg H₂O/l to the patient which creates uncertainty for the use in certain conditions. For example, most schools of thought believe that HME's are not suitable for patients undergoing long term sedation and for use with paediatrics or patients with other certain indications. Despite some drawbacks HME's have become widely used particularly for adults and short intravenous sedations.

It is an object of the invention which is the subject of this patent application to address the aforementioned drawbacks of active and passive humidification. The combining of passive humidification with meaningful active humidification poses many challenges. There have been numerous attempts previously to solve these challenges, but all have only had limited success. There is therefore a need for an improved active and passive humidification device for mounting in patient ventilation circuits.

WO2018/035579 describes a respiratory pressure therapy system with a nebulising humidifier. Moreover, WO9119527, BE1003716, US5109471 and US5769071 describe HME units in a respiratory system.

The present invention also relates to a sedation device for insertion between the ET-tube and the Y-piece of a ventilator circuit to vaporize and reflect/conserve a volatile sedative. This type of sedation device essentially comprises a housing having an interior separated by an activated carbon filter into a ventilator chamber and a patient chamber. The ventilator chamber connects to a ventilator and the patient chamber connects to the patient. An evaporator mounted in the patient chamber is operable to deliver a volatile sedative to the patient during breathing. Each time the patient exhales sedative, heat and moisture from the breathing gas is captured by the filter for subsequent release back into the airstream during the next inspiration. Thus, in addition to reflecting volatile sedative, the filter forms a passive humidifier which maintains a level of humidity in air inhaled by the patient. The capture and return of heat and moisture to the patient reduces the chance of adverse conditions associated with artificial ventilation occurring, such as the drying out of lung tissue or patient secretions, as well as considerably reducing the amount of respiratory heat lost by the body. The passive humidification provided by the sedation device can maintain an air temperature of about 33°C and relative humidity of about 79%. However, the ideal amount of humidification for mechanically ventilated patients is an air temperature of 37°C with 44 mg H₂O/L absolute humidity resulting in 100% relative humidity.

The present invention is directed towards addressing the limitations of existing active and passive offerings which attempt to address these issues. The present invention is, however, limited by the appended claims.

### Summary of the Invention

According to the invention, there is provided an active and passive humidification device for mounting in a patient ventilation circuit, the device, including:
a housing having a ventilator chamber and an associated patient chamber communicating with the ventilator chamber through a gas permeable filter mounted between the ventilator chamber and the patient chamber,
said filter forming a passive humidifier which is operable to capture and reflect heat and moisture received from a patient back to said patient,
the ventilator chamber having a ventilator connection port for connection to a ventilator,
the patient chamber having a patient connector port for connection to a patient breathing tube,
an humidity generating device mounted on the housing and being operable to discharge moisture into the patient chamber,
characterised in that a heater is mounted within the patient chamber, a temperature sensor is mounted within the patient chamber, the humidity generating device, the heater and the temperature sensor being connected to an associated controller which is operable to regulate operation of the heater and the humidity generating device to maintain air at a desired temperature and humidity for delivery from the patient chamber to a patient.

Advantageously the active humidifier device operates in tandem with the passive humidification facilitated by the filter to maintain air at a desired optimum humidification for delivery to a patient.

In one embodiment of the invention an air flow sensor is mounted within the housing to detect movement of air through the housing, the air flow sensor being connected to the controller which regulates operation of the humidity generating device such that the humidity generating device is switched on by the controller during a patient inhalation and switched off by the controller during a patient exhalation.

In another embodiment of the invention the controller is operable to switch off the humidity generating device at a preset time interval before the end of a patient inhalation.

In another embodiment of the invention the heater comprises a heater plate mounted within the patient chamber.

In another embodiment of the invention the heater plate is shaped to correspond to the contour of an outer wall of the patient chamber.

In another embodiment of the invention the heater plate is mounted against the outer wall of the patient chamber.

In another embodiment of the invention the heater plate is mounted spaced-apart from the outer wall of the patient chamber.

In another embodiment of the invention the heater plate has outwardly projecting fins on an inner face of the heater plate.

In another embodiment of the invention the heater is a ceramic heater plate.

In another embodiment of the invention an insulation element is mounted on the patient chamber outside the heater plate.

In another embodiment of the invention the insulation element is mounted on an outside face of the outer wall of the patient chamber.

In another embodiment of the invention the insulation element has a wiring conduit passing through the insulation element.

In another embodiment of the invention the patient chamber has a bottom wall which slopes downwardly towards the patient connector port for delivery of any moisture collected in the patient chamber towards the patient connector port.

In another embodiment of the invention a cowl is mounted about the air flow sensor, the cowl having an opening facing an outer end of the ventilator connector port.

In another embodiment of the invention the air flow sensor is operable to detect the direction of air flow through the housing.

In another embodiment of the invention the air flow sensor is operable to detect the volume of air flow through the housing.

In another embodiment of the invention the air flow sensor is mounted within the ventilator connection port or the ventilator chamber.

In another embodiment of the invention an evaporator is mounted within the patient chamber for delivery of a volatile anaesthetic into the patient chamber.

In another embodiment of the invention the humidity generating device is demountably engagable with the housing.

In another embodiment of the invention the humidity generating device is integral with the housing.

In another embodiment of the invention the humidity generating device is mounted within the patient chamber.

In another embodiment of the invention the humidity generating device is mounted at or adjacent the patient connector port.

### Brief Description of the Drawings

The invention will be more clearly understood by the following description of some embodiments thereof, given by way of example only, with reference to the accompanying drawings, in which:
Fig. 1 is a perspective view of a patient ventilating device according to the invention;
Fig. 2 is a sectional perspective view of the device shown in Fig. 1;
Fig. 3 is an elevational view of the device shown in Fig. 1;
Fig. 4 is a sectional elevational view of the device shown in Fig. 1;
Fig. 5 is a schematic illustration of a patient ventilating system incorporating the device shown in Fig. 1;
Fig. 6 is a perspective view of another patient ventilating device according to a further embodiment of the invention;
Fig. 7 is a sectional perspective view of the device shown in Fig. 6;
Fig. 8 is an elevational view of the device shown in Fig. 6;
Fig. 9 is a sectional elevational view of the device shown in Fig. 6,
Fig. 10 is a schematic sectional elevational view of another patient ventilating device;
Fig. 11 is a sectional elevational view of another patient ventilating device according to the invention;
Fig. 12 is an enlarged detail sectional elevational view showing portion of the device of Fig. 11;
Fig. 13 is an elevational view of a sedation device according to another embodiment of the invention;
Fig. 14 is an exploded, partially sectioned, elevational view of the sedation device shown in Fig. 13;
Fig. 15 is a sectional elevational view of a sedation device according to another embodiment of the invention;
Fig. 16 is a sectional elevational view of a sedation device according to another embodiment of the invention;
Fig. 17 is a sectional elevational view of a sedation device according to another embodiment of the invention;
Fig. 18 is a sectional elevational view of a sedation device according to another embodiment of the invention;
Fig. 19 is a sectional elevational view of a sedation device according to another embodiment of the invention, and
Fig. 20 is a sectional elevational view of a sedation device according to another embodiment of the invention.

### Detailed Description of the Preferred Embodiments

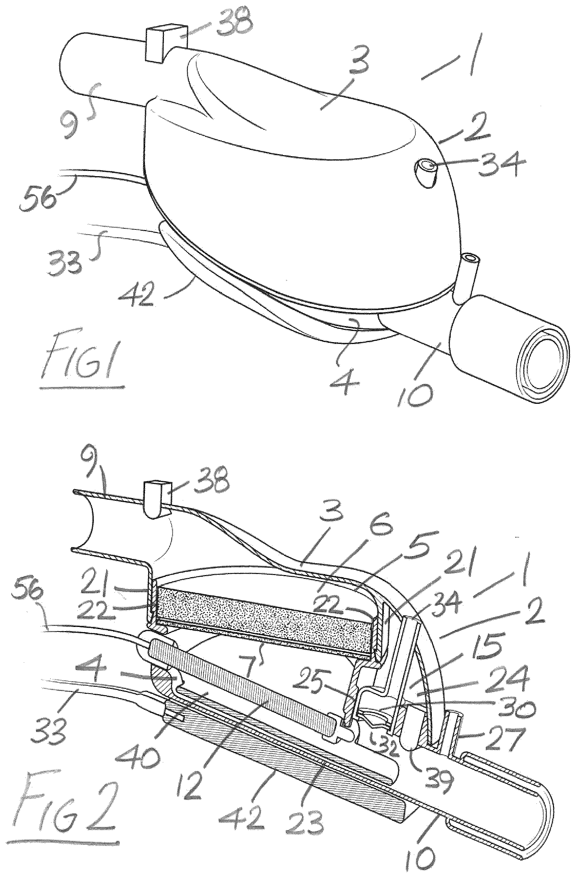

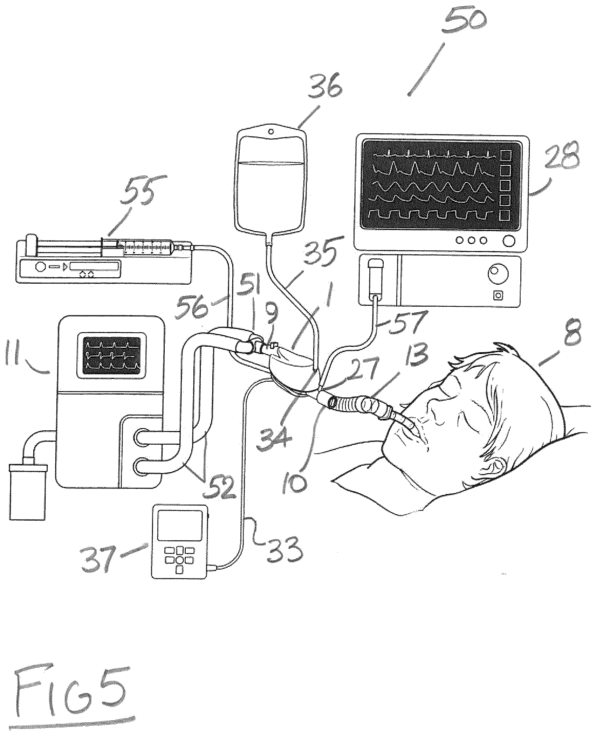

Referring to the drawings, and initially to Figs. 1 to 5 thereof, there is shown an active and passive humidification device for mounting in a patient ventilation circuit according to the invention indicated generally by the reference numeral 1. The device 1 comprises a housing 2 having a ventilator chamber 3 and an associated patient chamber 4 communicating with the ventilator chamber 3 through a gas permeable filter 5 mounted between the ventilator chamber 3 and the patient chamber 4. The filter 5 comprises a carbon filter element 6 and an associated antibacterial filter element 7 mounted against a bottom face of the carbon filter element 6. This filter 5 forms a passive humidifier which is operable in use to capture and reflect heat and moisture received from a patient 8 (Fig. 5) back to the patient 8.

The ventilator chamber 3 has a ventilator connector port 9 for connection to a ventilator 11. The patient chamber 4 has a patient connector port 10 for connection to a patient breathing tube 13. An evaporator, in this case provided by a vaporising rod 12, is mounted within the patient chamber 4. A humidity generating device 15 is integrated in the housing 2 and is operable to control the humidity of air delivered from the patient chamber 4 through the patient connector port 10 to a patient 8.

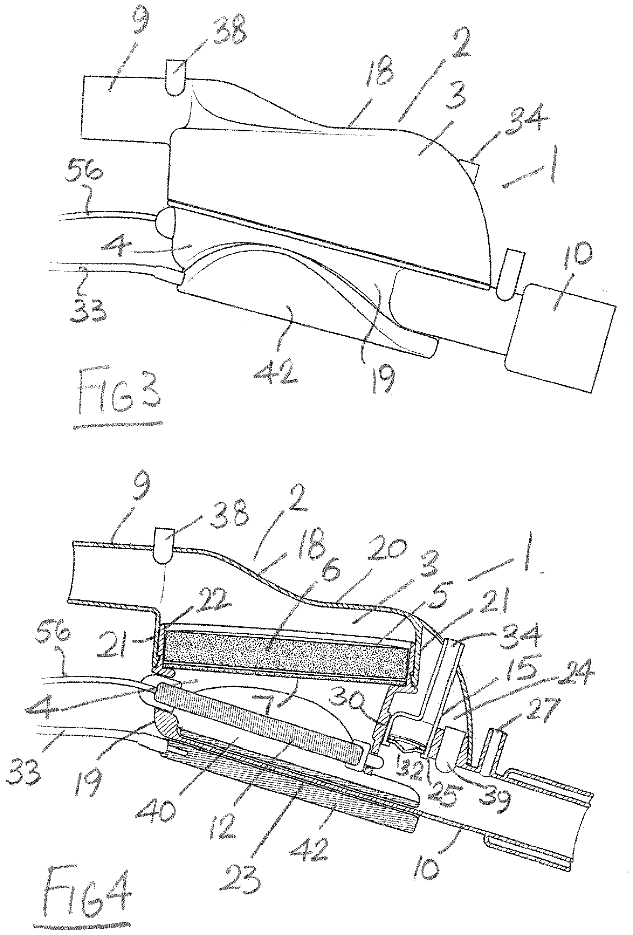

Referring in particular to Fig. 3 and Fig. 4, the housing 2 may conveniently be made of plastics material and is provided in two parts which secure together to form the housing 2. These two parts comprise a ventilator chamber housing 18 and an associated patient chamber housing 19. The ventilator chamber housing 18 has a top wall 20 with a downwardly depending peripheral skirt 21 which slidably engages and fits outside an associated upstanding peripheral rim 22 on the patient chamber housing 19, the chamber housings 18, 19 being welded together or adhesively secured together upon assembly. It will be noted that an outer or bottom wall 23 of the patient chamber 4 is conveniently sloped to drain any moisture collected in the patient chamber 4 towards the patient connector port 10.

A humidifier mounting chamber 24 is formed in the housing 2 adjacent the patient connector port 10. The humidity generating device 15 is housed within the humidifier mounting chamber 24. A passageway 25 connects between the humidifier chamber 24 and the patient connector port 10 within which the humidity generating device 15 is mounted. A gas sampling port 27 projects outwardly of the patient connector port 10 and can be connected to a gas sampling device 28 for measuring the anaesthetic content delivered to the patient 8.

The humidifier device 15 comprises a housing 30 mounted in the passageway 25 and having a vapour outlet 32 at a bottom of the housing 30. A water inlet 34 at a top of the housing 30 allows delivery of water or saline into the housing 30 for vaporisation and subsequent discharge of water vapour or saline vapour through the vapour outlet 32 into the patient connector port 10. A water or saline supply can be provided either by way of a refillable reservoir on the sedation device 1, or more preferably by a water feed line 35 from a remote water supply or reservoir 36 as shown in Fig. 5.

The humidity generating device 15 is connected to an associated controller 37 which regulates operation of the humidity generating device 15. An associated air flow sensor 38 is mounted at the ventilator connector port 9 and is also connected to the controller 37. A temperature sensor 39 is mounted at the patient connector port 10 and connected to the controller 37. The temperature sensor 39 is positioned at an exit from the patient chamber 4 and is located downstream of the humidity generating device 15, that is between the humidity generating device 15 and the patient 8 to ensure an accurate reading of the temperature of the humidified air delivered to the patient 8 upon inhalation.

The patient chamber 4 is fitted with a specially designed ceramic heater plate 40 which conforms and fits snugly to an inner face of the curved bottom wall 23 of the patient chamber 4. The heater plate 40 is shaped to correspond to the internal contour of the bottom wall 23, nesting against the bottom wall 23. The heater plate 40 is connected to the controller 37 and cooperates with the sensors 38, 39 and the humidity generating device 15. The heater plate 40 has a relatively large surface area to facilitate rapid and even temperature control within the patient chamber 4. The patient chamber 4 may be made from a heat-resistant, but biocompatible material.

An external surface of the patient chamber 4 is fitted with an insulation element 42 to protect the patient, medical professionals and the environment from the heat generated within the patient chamber 4 by the ceramic heater plate 40. The insulation element 42 is suitably shaped to match the contour of an exterior surface of the patient chamber 4 and to support ergonomic handling of the device 1. Conveniently, the insulation element 42 may facilitate the connection of the sensors 38, 39 and humidity generating device 15 to the controller 37 (or may have a Bluetooth sensor) and will incorporate all the wiring 33 connecting the heater plate 40, humidity generating device 15 and the sensors 38, 39 to the controller 37.

By controlling operation of the heater plate 40 and the humidity generating device 15, a range of temperatures and moisture outputs of 33°C and 35.5 mg H₂O/I up to 37°C and 44 mg H₂O/I can be achieved.

The airflow sensor 38 mounted in the ventilator chamber 3 will detect the beginning and end of inspiration and the controller 37 will stop the delivery of moisture at a predetermined point before the end of inspiration. This prevents the patient exhaling excess moisture onto the filter 5.

The air flow sensor 38 is operable to sense the direction of air flow through the device 1. It also measures the volume of air moving through the device 1 upon each inhalation and exhalation by the patient 8. The air flow sensor 38 will cooperate with the controller 37 to determine the flow rate and the duration of flow and will use its learned history to instruct the humidity generating device 15 to deliver the correct amount of moisture to achieve the required relative humidity at the preset temperature.

Referring in particular to Fig. 5, the device 1 is shown incorporated in a patient ventilating system, indicated generally by the reference numeral 50. The ventilator connector port 9 is connected to the ventilator 11 by means of a Y-connector piece 51 and associated ventilator tubes 52. An endotracheal tube 13 connects between the patient connector port 10 and a patient 8. An anaesthetic delivery device 55 connects via a feed line 56 with the vaporising rod 12 within the device 1. The gas sampling port 27 of the device 1 connects through a gas sampling line 57 with the gas sampling device 28 which has a display screen to show measured anaesthetic concentration.

In use, the device 1 is mounted in the usual way in a ventilating system 50 between a ventilator 11 and a patient 8. A volatile sedative at a selected dosage is delivered into the air within the patient chamber 4 by the evaporator 12 for inhalation by the patient 8. As the patient 8 exhales the exhaled air passes through the sedation device 1 and sedative, heat and moisture is captured by the filter 5 for subsequent release back into the air upon the next inhalation by the patient 8. Thus the filter 5 provides a passive humidification of the air breathed by the patient 8. In this way the sedation device will operate at an air temperature of about 33°C with 28 mg H₂O/L absolute humidity resulting in 79% relative humidity. In addition to this passive humidification by operation of the humidity generating device 15 an optimum air condition, providing an air temperature of 37°C with mg H₂O/L absolute humidity resulting in 100% relative humidity, can be achieved and maintained.

The controller 37 controls operation of the humidity generating device 15 and the heating plate 40. The air flow sensor 38 senses whether air is travelling to or from the ventilator 11. The controller 37 regulates operation of the humidity generating device 15 such that it is switched on by the controller 37 during a patient inhalation and switched off by the controller 37 during a patient exhalation. It will be noted that the controller 37 switches off the active humidity generating device 15 at a preset time interval before the end of a patient inhalation. The heater plate 40 is operable to control the temperature of air delivered to the patient 8. The temperature sensor 39 senses air temperature at the patient connector port 10 and the controller 137 controls operation of the heater plate 40 to achieve the desired air temperature. Further, in response to the sensed temperature, the controller 37 operates the humidity generating device 15 to achieve a desired relative humidity in the air delivered to the patient 8. Typically, this will be at or approaching 100% relative humidity and may be controlled within a desired range of 85%-100% relative humidity at a controlled constant temperature ranging up to 37°C. More preferably the humidity is controlled to between 95% - 100% relative humidity and most preferably at about 95% relative humidity which advantageously avoids rainout and possible excess moisture build-up.

The controller 37 uses the inputs from the air flow sensor 38 and the temperature sensor 39 to regulate operation of the heater plate 40 and the humidity generating device 15 to maintain air delivered to the patient 8 at a desired temperature and humidity.

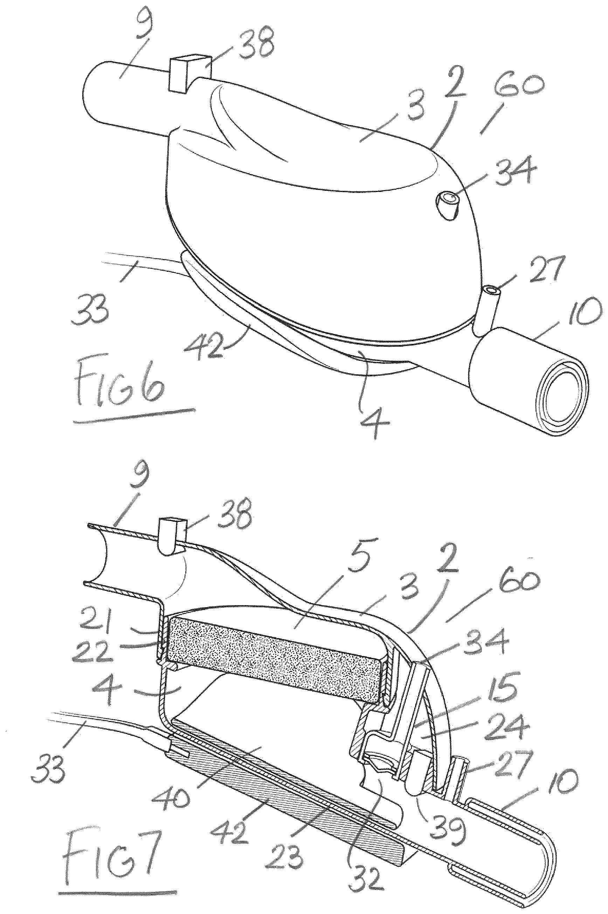

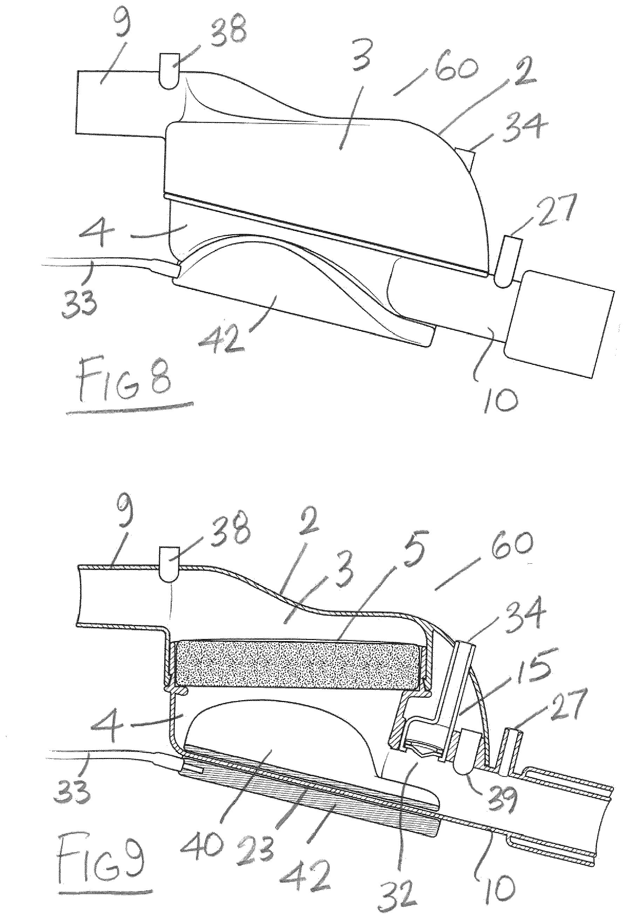

Referring now to Figs. 6 to 9, there is shown another active and passive humidification device for mounting in a patient ventilation circuit 50 according to another embodiment of the invention indicated generally by the reference numeral 60. Parts similar to those described previously are assigned the same reference numerals. This is largely similar to the device 1 described previously, however, in this case there is no evaporator incorporated in the device 60 which functions as a device with both passive and active humidification of air delivered between the ventilator 11 and the patient 8.

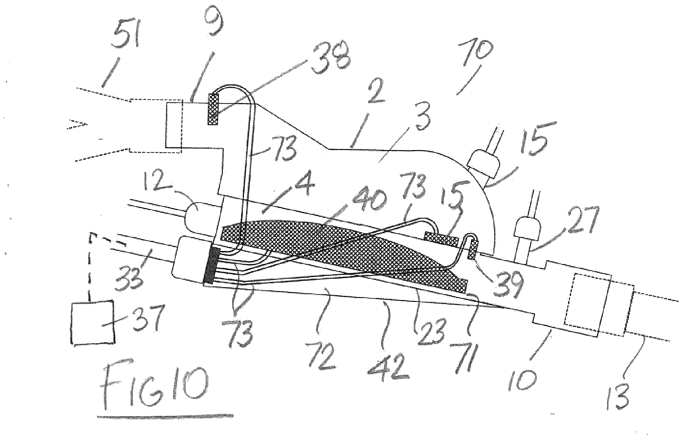

Referring now to Fig. 10, there is shown another active and passive humidification device for mounting in a patient ventilation circuit according to a further embodiment of the invention, indicated generally by the reference numeral 70. Parts similar to those described previously are assigned the same reference numerals. In this case, it will be noted that the ceramic heater plate 40 is spaced-apart from the bottom wall 23 of the patient chamber 4 leaving a gap 71 between the ceramic heater plate 40 and the bottom wall 23 which further insulates the heater plate 40 from the exterior of the device 70. It will be noted also that the insulation element 42 has a wiring conduit 72 passing through the insulation element 42 to allow through passage of wiring 33, 73 connecting the humidity generating device 15, sensors 38, 39 and heater plate 40 to the controller 37.

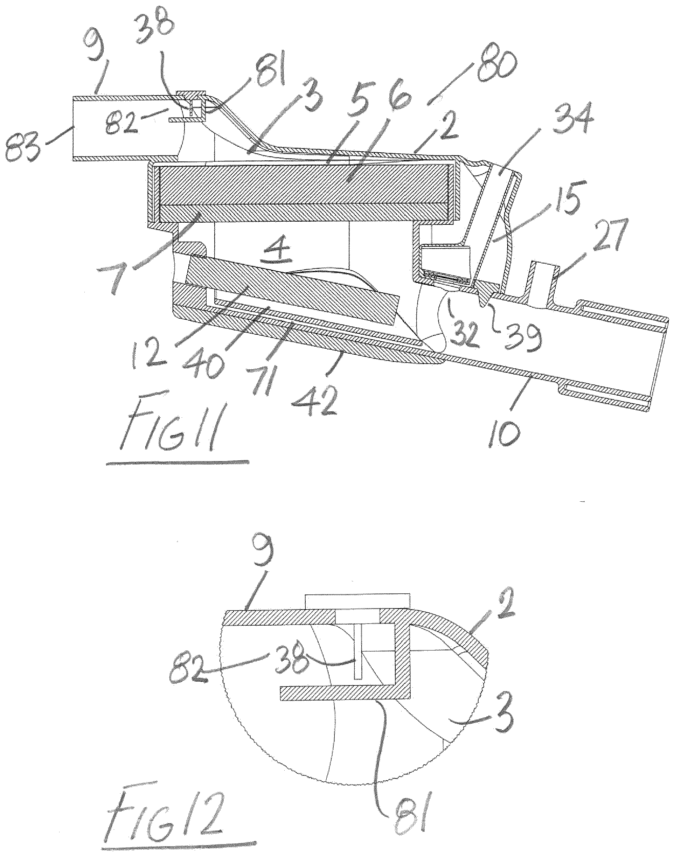

Referring now to Fig. 11 and Fig. 12, there is shown another active and passive humidification device for mounting in a patient ventilation circuit 50 according to another embodiment of the invention, indicated generally by the reference numeral 80. Parts similar to those described previously are assigned the same reference numerals. In this case a cowl 81 is mounted about the air flow sensor 38. The cowl 81 has an opening 82 facing an outer end 83 of the ventilator connector port 9. Thus, air flow through the device 80 towards the ventilator 11, that is when the patient exhales, passes around the cowl 81 and is shielded from the air flow sensor 38. That is, the air flow sensor is sheltered from the exhalation. The air flow sensor 38 is exposed to air flow travelling from the ventilator 11 to the patient 8, that is when the patient 8 inhales, by virtue of the opening 82.

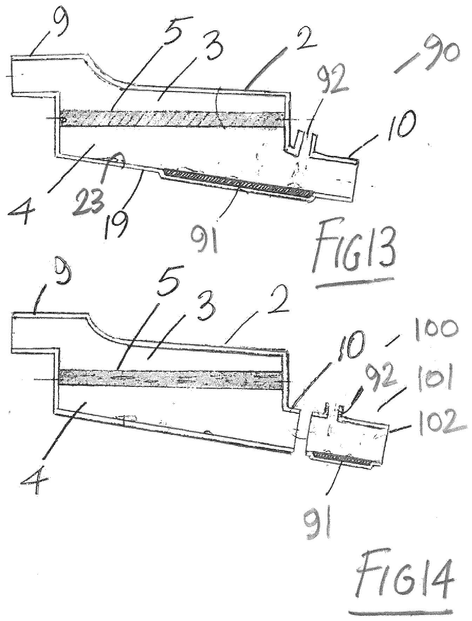

Referring now to Fig. 13, there is illustrated an active and passive humidification device for mounting in a patient ventilation circuit according to another embodiment of the invention, indicated generally by the reference numeral 90. Parts similar to those described previously are assigned the same reference numerals. In this case the humidity generating device is formed by a heating plate 91 mounted within the device 90 within the patient chamber 4 on an inside face of the bottom wall 23 of the patient chamber housing 19. An associated water inlet port 92 on a side of the patient connector port 10 can be connected up to a water supply for delivery of water into the patient chamber 4 where it is heated and vaporised by means of the heating plate 91.

Referring now to Fig. 14, there is illustrated an active and passive humidification device for mounting in a patient ventilation circuit according to another embodiment of the invention, indicated generally by the reference numeral 100. Parts similar to those described previously are assigned the same reference numerals. In this case a demountable humidity generating device 101 is provided having a tubular housing 102 which is engageable with the patient connector port 10 to mount the humidity generating device 101 on the housing 2. The heating plate 91 is mounted at the bottom of the housing 102 beneath the water inlet port 92.

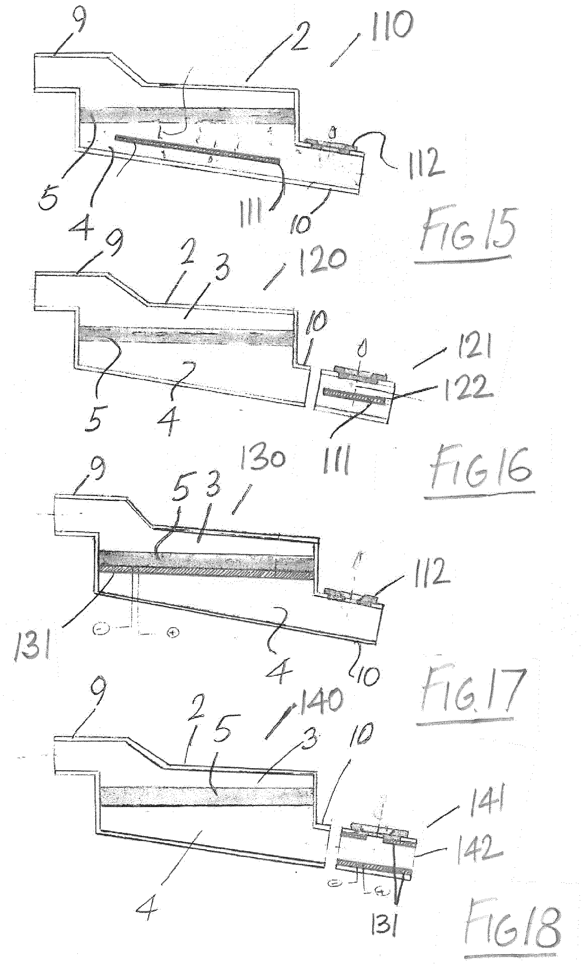

Referring now to Fig. 15, there is illustrated another active and passive humidification device for mounting in a patient ventilation circuit according to another embodiment of the invention, indicated generally by the reference numeral 110. Parts similar to those described previously are assigned the same reference numerals. In this case a heated plate 111 is mounted within the patient chamber 4 and cooperates with a humidity generating device 112 mounted on the patient connector port 10 to humidify air within the patient chamber 4.

Referring now to Fig. 16, there is illustrated an active and passive humidification device for mounting in a patient ventilation circuit according to another embodiment of the invention, indicated generally by the reference numeral 120. Parts similar to those described previously are assigned the same reference numerals. This is similar to the device shown in Fig. 15. In this case a demountable humidity generating device 121 is provided having a tubular housing 122 which is demountably engageable with the patient connector port 10. The heated plate 111 is mounted within the housing 122 of the humidity generating device 121 to heat water vapour generated by the humidity generating device 121.

Referring now to Fig. 17, there is illustrated an active and passive humidification device for mounting in a patient ventilation circuit according to another embodiment of the invention, indicated generally by the reference numeral 130. Parts similar to those described previously are assigned the same reference numerals. In this case the humidity generating device is formed by a carbon resistor panel 131 mounted within the patient chamber 4 at an underside of the filter 5. The carbon resistor panel 131 is connected to an electrical supply for heating the carbon resistor panel 131 for heating vaporised water generated by the humidity generating device 112 mounted on the patient connector port 10 and delivered into the patient chamber 4.

Referring now to Fig. 18, there is illustrated an active and passive humidification device for mounting in a patient ventilation circuit according to another embodiment of the invention, indicated generally by the reference numeral 140. Parts similar to those described previously are assigned the same reference numerals. This is similar to the arrangement shown in Fig. 17 but in this case a humidity generating device 141 is demountably engagable with the patient connector port 10. The humidity generating device 141 has a tubular housing 142 through which water passes and is vaporised to be heated within the housing 142 by heated carbon resistor panels 131 on an inside face of the housing 142.

It will be appreciated that any suitable humidity generating device may be employed in the devices of the invention, including for example a perforated piezoelectric plate, an ultrasonic vibrating plate, a syringe vaporiser for squeezing water through a perforated plate, an injection or infusion device or a heated wick.

The device and method of the present invention provides a number of advantages over existing active and passive humidification devices, including:
Provision of incremental humidification delivery above the capability of a standard HME device up to the limit of 37 °C and 44 mg H₂O/I.
Ability to control the delivery of Incremental humidification at a range of temperatures above the capability of the HME device.
Provides a method for addressing the additional moisture which is likely to build up in the device.
Provision of a method of dealing with the many modes of ventilation other than volume control.
Provision of a heating method which is capable of reaching the temperatures required but which is safe in the oxygen-rich environment.
Providing a method which prevents the heat of the device affecting the patient, the medical professional or the use environment.
Provision of a method which delivers safe and discrete power and communication between the major components of the device.
Provision of a method to prevent excess moisture being exhaled on to the sensitive filter.
Provision of a method to deliver the heat and moisture over the duration of inhalation but ceasing at a predetermined point before the end of inhalation.
Positioning an air flow sensor at the ventilator connector port or in the ventilator chamber provides a method of measuring air flow in a safe and clean environment free from moisture, secretions and anaesthetic.

The terms "comprise" and "include", and any variations thereof required for grammatical reasons, are to be considered as interchangeable and accorded the widest possible interpretation.

The invention is not limited to the embodiments hereinbefore described but may be varied in construction and detail within the scope of the appended claims.

## Claims

1. An active and passive humidification device (1) for mounting in a patient ventilation circuit (50), the device (1) including:
a housing (2) having a ventilator chamber (3) and an associated patient chamber (4) communicating with the ventilator chamber (3) through a gas permeable filter (5) mounted between the ventilator chamber (3) and the patient chamber (4),
said filter (5) forming a passive humidifier which is operable to capture and reflect heat and moisture received from a patient (8) back to said patient (8),
the ventilator chamber (3) having a ventilator connection port (9) for connection to a ventilator (11),
the patient chamber (4) having a patient connector port (10) for connection to a patient breathing tube (13),
a humidity generating device (15) mounted on the housing (2) and being operable to discharge moisture into the patient chamber (4),
wherein a heater (40) is mounted within the patient chamber (4), a temperature sensor (39) is mounted within the patient chamber (4), the humidity generating device (15), the heater (40) and the temperature sensor (39) being connected to an associated controller (37) which is operable to regulate operation of the heater (40) and the humidity generating device (15) to maintain air at a desired temperature and humidity for delivery from the patient chamber (4) to a patient (8).

2. The device (1) as claimed in claim 1 wherein an air flow sensor (38) is mounted within the housing (2) to detect movement of air through the housing (2), the air flow sensor (38) being connected to the controller (37) which regulates operation of the humidity generating device (15) such that the humidity generating device (15) is switched on by the controller (37) during a patient inhalation and switched off by the controller (37) during a patient exhalation.

3. The device (1) as claimed in Claim 2, wherein the controller (37) is operable to switch off the humidity generating device (15) at a preset time interval before the end of a patient inhalation.

4. The device (1) as claimed in any preceding claim, wherein the heater comprises a heater plate (40) mounted within the patient chamber (4), the heater plate (40) is shaped to correspond to the contour of an outer wall (23) of the patient chamber (4).

5. The device (1) as claimed in claim 4 wherein the heater plate (40) is mounted against the outer wall (23) of the patient chamber (4).

6. The device (1) as claimed in claim 4 or claim 5 wherein the heater plate (40) is mounted spaced-apart from the outer wall (23) of the patient chamber (4).

7. The device (1) as claimed in any one of claims 4 to 6 wherein the heater plate (40) has outwardly projecting fins on an inner face of the heater plate (40).

8. The device (1) as claimed in any preceding claim wherein the heater is a ceramic heater plate (40).

9. The device (1) as claimed in any one of claims 4 to 8 wherein an insulation element (42) is mounted on the patient chamber (4) outside the heater plate (40).

10. The device (1) as claimed in claim 9 wherein the insulation element (42) has a wiring conduit (72) passing through the insulation element (42).

11. The device (1) as claimed in any preceding claim, wherein the patient chamber (4) has a bottom wall (23) which slopes downwardly towards the patient connector port (10) for delivery of any moisture collected in the patient chamber (4) towards the patient connector port (10).

12. The device (1) as claimed in any one of claims 2 to 11 wherein a cowl (81) is mounted about the air flow sensor (38), the cowl (81) having an opening (82) facing an outer end (83) of the ventilator connector port (9).

13. The device (1) as claimed in any one of claims 2 to 12 wherein the air flow sensor (38) is operable to detect the direction of air flow through the housing (2).

14. The device (1) as claimed in any one of claims 2 to 13 wherein the air flow sensor (38) is operable to detect the volume of air flow through the housing (2).

15. The device (1) as claimed in any one of claims 2 to 14 wherein the air flow sensor (38) is mounted within the ventilator connection port (9) or the ventilator chamber (3).

16. The device (1) as claimed in any preceding claim wherein an evaporator (12) is mounted within the patient chamber (4) for delivery of a volatile anaesthetic into the patient chamber (4).

## Patentansprüche

1. Aktive und passive Befeuchtungsvorrichtung (1) zum Montieren in eines Patientenbeatmungskreislaufs (50), wobei die Vorrichtung (1) einschließt:
ein Gehäuse (2), das eine Beatmungskammer (3) und eine zugehörige Patientenkammer (4) aufweist, die mit der Beatmungskammer (3) durch einen gasdurchlässigen Filter (5) hindurch kommunizieren, der zwischen der Beatmungskammer (3) und der Patientenkammer (4) montiert ist,
wobei der Filter (5) einen passiven Befeuchter ausbildet, der betriebsfähig ist, um Wärme und Feuchtigkeit, die von einem Patienten (8) empfangen werden, zurück an den Patienten (8) zu erfassen und zu reflektieren,
wobei die Beatmungskammer (3) einen Beatmungsverbindungsanschluss (9) für eine Verbindung an eine Beatmungsmaschine (11) aufweist,
wobei die Patientenkammer (4) einen Patientenverbindungsanschluss (10) für die Verbindung an einen Patientenatemschlauch (13) aufweist,
eine Feuchtigkeitserzeugungsvorrichtung (15), die auf dem Gehäuse (2) montiert ist und betriebsfähig ist, um Feuchtigkeit in die Patientenkammer (4) auszustoßen,
wobei ein Heizelement (40) innerhalb der Patientenkammer (4) montiert ist, wobei ein Temperatursensor (39) innerhalb der Patientenkammer (4) montiert ist, wobei die Feuchtigkeitserzeugungsvorrichtung (15), das Heizelement (40) und der Temperatursensor (39) mit einer zugehörigen Steuerung (37) verbunden sind, die betriebsfähig ist, um einen Betrieb des Heizelements (40) und der Feuchtigkeitserzeugungsvorrichtung (15) zu regulieren, um Luft bei einer gewünschten Temperatur und Feuchtigkeit für eine Abgabe von der Patientenkammer (4) zu einem Patienten (8) zu halten.

2. Vorrichtung (1) nach Anspruch 1, wobei ein Luftströmungssensor (38) innerhalb des Gehäuses (2) montiert ist, um eine Bewegung von Luft durch das Gehäuse (2) hindurch zu erkennen, wobei der Luftströmungssensor (38) mit der Steuerung (37) verbunden ist, die den Betrieb der Feuchtigkeitserzeugungsvorrichtung (15) derart reguliert, dass die Feuchtigkeitserzeugungsvorrichtung (15) während einer Patienteneinatmung durch die Steuerung (37) eingeschaltet und während einer Patientenausatmung durch die Steuerung (37) ausgeschaltet wird.

3. Vorrichtung (1) nach Anspruch 2, wobei die Steuerung (37) betriebsfähig ist, um die Feuchtigkeitserzeugungsvorrichtung (15) in einem voreingestellten Zeitintervall vor dem Ende einer Patienteneinatmung auszuschalten.

4. Vorrichtung (1) nach einem der vorstehenden Ansprüche, wobei das Heizelement eine Heizplatte (40) umfasst, die innerhalb der Patientenkammer (4) montiert ist, wobei die Heizplatte (40) geformt ist, um der Kontur einer Außenwand (23) der Patientenkammer (4) zu entsprechen.

5. Vorrichtung (1) nach Anspruch 4, wobei die Heizplatte (40) gegen die Außenwand (23) der Patientenkammer (4) montiert ist.

6. Vorrichtung (1) nach Anspruch 4 oder 5, wobei die Heizplatte (40) beabstandet von der Außenwand (23) der Patientenkammer (4) montiert ist.

7. Vorrichtung (1) nach einem der Ansprüche 4 bis 6, wobei die Heizplatte (40) nach außen vorstehende Rippen auf einer Innenfläche der Heizplatte (40) aufweist.

8. Vorrichtung (1) nach einem der vorstehenden Ansprüche, wobei das Heizelement eine Keramikheizplatte (40) ist.

9. Vorrichtung (1) nach einem der Ansprüche 4 bis 8, wobei ein Isolierelement (42) auf der Patientenkammer (4) außerhalb der Heizplatte (40) montiert ist

10. Vorrichtung (1) nach Anspruch 9, wobei das Isolationselement (42) eine Verdrahtungsleitung (72) aufweist, die durch das Isolationselement (42) hindurch verläuft.

11. Vorrichtung (1) nach einem der vorstehenden Ansprüche, wobei die Patientenkammer (4) eine Bodenwand (23) aufweist, die für die Abgabe einer beliebigen Feuchtigkeit, die in der Patientenkammer (4) gesammelt wird, zu dem Patientenverbinderanschluss (10) hin nach unten zu dem Patientenverbinderanschluss (10) neigt.

12. Vorrichtung (1) nach einem der Ansprüche 2 bis 11, wobei eine Haube (81) um den Luftströmungssensor (38) herum montiert ist, wobei die Haube (81) eine Öffnung (82) aufweist, die einem äußeren Ende (83) des Beatmungsverbinderanschlusses (9) zugewandt ist.

13. Vorrichtung (1) nach einem der Ansprüche 2 bis 12, wobei der Luftströmungssensor (38) betriebsfähig ist, um die Richtung der Luftströmung durch das Gehäuse (2) hindurch zu erkennen.

14. Vorrichtung (1) nach einem der Ansprüche 2 bis 13, wobei der Luftströmungssensor (38) betriebsfähig ist, um das Volumen an Luftströmung durch das Gehäuse (2) hindurch zu erkennen.

15. Vorrichtung (1) nach einem der Ansprüche 2 bis 14, wobei der Luftströmungssensor (38) innerhalb des Beatmungsverbindungsanschlusses (9) oder der Beatmungskammer (3) montiert ist.

16. Vorrichtung (1) nach einem der vorstehenden Ansprüche, wobei ein Verdampfer (12) innerhalb der Patientenkammer (4) für die Abgabe eines Inhalationsanästhetikums in die Patientenkammer (4) montiert ist.

## Revendications

1. Dispositif d'humidification actif et passif (1) destiné à être monté dans un circuit de ventilation de patient (50), le dispositif (1) comportant :
un boîtier (2) ayant une chambre de ventilateur (3) et une chambre de patient associée (4) communiquant avec la chambre de ventilateur (3) à travers un filtre perméable aux gaz (5) monté entre la chambre de ventilateur (3) et la chambre de patient (4),
ledit filtre (5) formant un humidificateur passif qui est exploitable pour capturer et réfléchir la chaleur et l'humidité reçue d'un patient (8) vers ledit patient (8),
la chambre de ventilateur (3) ayant un orifice de connexion de ventilateur (9) pour le raccordement à un ventilateur (11),
la chambre de patient (4) ayant un orifice de connexion de patient (10) pour le raccordement à un tube respiratoire du patient (13),
un dispositif de production d'humidité (15) monté sur le boîtier (2) et pouvant fonctionner pour décharger l'humidité dans la chambre de patient (4),
dans lequel un dispositif de chauffage (40) est monté à l'intérieur de la chambre de patient (4), un capteur de température (39) est monté à l'intérieur de la chambre de patient (4), le dispositif de production d'humidité (15), le dispositif de chauffage (40) et le capteur de température (39) étant connectés à un dispositif de commande associé (37) qui est exploitable pour réguler le fonctionnement du dispositif de chauffage (40) et du dispositif de production d'humidité (15) pour maintenir l'air à une température et à l'humidité souhaitées pour distribuer de la chambre de patient (4) à un patient (8).

2. Dispositif (1) selon la revendication 1, dans lequel un capteur de flux d'air (38) est monté à l'intérieur du boîtier (2) pour détecter un mouvement de l'air à travers le boîtier (2), le capteur de flux d'air (38) étant relié au dispositif de commande (37) qui régule le fonctionnement du dispositif de production d'humidité (15) de telle sorte que le dispositif de production d'humidité (15) est activé par le dispositif de commande (37) pendant une inhalation du patient et éteint par le dispositif de commande (37) pendant une expiration du patient.

3. Dispositif (1) selon la revendication 2, dans lequel le dispositif de commande (37) peut être actionné pour éteindre le dispositif de génération d'humidité (15) à un intervalle de temps prédéfini avant la fin d'une inhalation de patient.

4. Dispositif (1) selon l'une quelconque revendication précédente, dans lequel le dispositif de chauffage comprend une plaque chauffante (40) montée dans la chambre de patient (4), la plaque chauffante (40) est façonnée pour correspondre au contour d'une paroi extérieure (23) de la chambre de patient (4).

5. Dispositif (1) selon la revendication 4, dans lequel la plaque chauffante (40) est montée contre la paroi extérieure (23) de la chambre de patient (4).

6. Dispositif (1) selon la revendication 4 ou 5, dans lequel la plaque chauffante (40) est montée espacée de la paroi extérieure (23) de la chambre de patient (4).

7. Dispositif (1) selon l'une quelconque des revendications 4 à 6, dans lequel la plaque chauffante (40) présente des ailettes faisant saillie vers l'extérieur sur une face intérieure de la plaque chauffante (40).

8. Dispositif (1) selon l'une quelconque revendication précédente, dans lequel le dispositif de chauffage est une plaque chauffante en céramique (40).

9. Dispositif (1) selon l'une quelconque des revendications 4 à 8, dans lequel un élément d'isolation (42) est monté sur la chambre de patient (4) à l'extérieur de la plaque chauffante (40).

10. Dispositif (1) selon la revendication 9, dans lequel l'élément d'isolation (42) a une conduite de câblage (72) passant à travers l'élément d'isolation (42).

11. Dispositif (1) selon l'une quelconque revendication précédente, dans lequel la chambre de patient (4) présente une paroi de fond (23) qui est inclinée vers le bas en direction de l'orifice de connexion de patient (10) pour distribuer toute humidité collectée dans la chambre de patient (4) vers l'orifice de connexion de patient (10).

12. Dispositif (1) selon l'une quelconque des revendications 2 à 11, dans lequel un capot (81) est monté autour du capteur de flux d'air (38), le capot (81) ayant une ouverture (82) faisant face à une extrémité extérieure (83) de l'orifice de connexion de ventilateur (9).

13. Dispositif (1) selon l'une quelconque des revendications 2 à 12, dans lequel le capteur de flux d'air (38) peut fonctionner pour détecter la direction du flux d'air à travers le boîtier (2).

14. Dispositif (1) selon l'une quelconque des revendications 2 à 13, dans lequel le capteur de flux d'air (38) peut fonctionner pour détecter le volume du flux d'air à travers le boîtier (2).

15. Dispositif (1) selon l'une quelconque des revendications 2 à 14, dans lequel le capteur de flux d'air (38) est monté à l'intérieur de l'orifice de connexion de ventilateur (9) ou de la chambre de ventilateur (3).

16. Dispositif (1) selon l'une quelconque revendication précédente, dans lequel un évaporateur (12) est monté à l'intérieur de la chambre de patient (4) pour l'administration d'un anesthésique volatil dans la chambre de patient (4).
